# EUROPEAN PATENT APPLICATION

(11) **EP 1 749 535 A1**
(43) Date of publication of application: **07.02.2007**
(21) Application number: 05729190.8
(22) Date of filing: 08.04.2005
(51) Int. Cl.: A61K 38/18, A61K 47/42, A61P 25/00

(54) **REMEDY FOR DIABETIC NEUROPATHY COMPRISING GELATIN GEL AS CARRIER**

(30) Priority: 09.04.2004 JP 2004116109
(71) Applicant: Nakamura, Jiro, Nagoya-shi, Aichi 4650054 (JP); Tabata, Yasuhiko, Uji-shi, Kyoto 611-0024 (JP); KAKEN PHARMACEUTICAL CO., LTD., Bunkyo-ku, Tokyo 113-8650 (JP)
(72) Inventor: NAKAMURA, Jiro, Nagoya-shi, Aichi 4650054 (JP); TABATA, Yasuhiko, Kyoto 6110024 (JP)
(74) Representative: HOFFMANN EITLE
(86) International application number: PCT/JP2005/007272
(87) International publication number: WO 2005/097166

(57) **Abstract**

The present invention provides a therapeutic agent for diabetic neuropathy capable of more effectively treating diabetic neuropathy, wherein a basic fibroblast growth factor is carried on a gelatin gel.

## Description

### Technical Field

The present invention relates to a therapeutic agent for diabetic neuropathy, which comprises a basic fibroblast growth factor (bFGF). More specifically, the present invention relates to a bFGF-carrying gelatin gel preparation for the treatment of diabetic neuropathy.

### Background Art

The number of diabetic patients in our country keeps on increasing, and the number of patients strongly suspected of suffering diabetes is 7.4 million, which is said to amount to 16.2 million including patients having undeniable possibility. In the medical care of diabetic patients, how to prevent incidence and progress of diabetic complications (retinopathy, nephropathy, neuropathy and the like) is an object of prime importance, where, from among the complications, neuropathy is considered to develop most frequently and from the early stages.

Diabetic neuropathy not only markedly degrades QOL (Quality of Life) of diabetic patients with a variety of clinical manifestations, but also immeasurably influences the life expectancy in cardiovascular autonomic neuropathy, and its prophylaxis and early treatment is important. While the incidence of diabetic neuropathy in diabetic patients heretofore reported varies depending on the diagnostic method and criteria employed, duration of diabetes, age, difference in the knowledge and experience of the examiner or technologist, it is generally said to be 30-40%. There are various groupings proposed for classification of neuropathy. Clinically, it is largely classified into polyneuropathy (sensorimotor neuropathy and autonomic neuropathy) and mononeuropathy (mixed spinal neuropathy and cranial neuropathy).

While it is needless to say that diabetic neuropathy develops and progresses from hyperglycemia as a basis, neurologic dysfunction is induced via various abnormalities caused by hyperglycemia. Representative abnormality derived from hyperglycemia includes abnormalities in metabolic factor, vascular factor and neurotrophic factor. In particular, the importance of metabolic factor and vascular factor and mutual relationship between them have been well studied heretofore, and it is considered that nerve cell dysfunction due to metabolic disorder caused by hyperglycemia and abnormal blood flow based on metabolic disorder are intricately entangled with the expression of neurologic dysfunction. As regards representative abnormal metabolism and abnormal neurotrophic factor, the following have been known.
1) enhancement of polyol metabolic activity: It is considered that the protein kinase C (PKC) activity and Na⁺/K⁺-ATPase activity are degraded via sorbitol accumulation and lack of myoinositol due to the enhancement of polyol metabolic activity in nerve cells, which in turn decreases the nerve conduction velocity, i.e., causes neuropathy.
2) abnormalities in PKC activity: It is considered that the promotion of PKC (particularly PKC-beta) activity in blood vessel cells is involved in abnormal blood flow and degraded PKC (particularly PKC-alpha) activity induces cell dysfunction in neurocytes, and it is assumed that the both are associated with the onset of neuropathy.
3) promotion of nonenzymatic glycation reaction: It is considered that advanced glycation endproducts (AGEs) due to a promoted nonenzymatic glycation reaction causes an abnormal blood flow in the intraneural blood vessel by acting on the intraneural vascular endothelial cells, while causing cell dysfunction by directly acting on neurocytes.
4) promotion of oxidative stress: It is considered that promoted production and degraded scavenging ability of active oxygen due to hyperglycemia cause promoted oxidative stress, as well as blood circulation disorder and cell dysfunction.
5) abnormalities in neurotrophic factor: A neurotrophic factor is an endogenous substance that maintains survival· differentiation·function of neuron and, as regards diabetic neuropathy, reports are mainly focused on nerve growth factor (NGF) and neurotrophin-3 (NT-3). It is considered that they improve neuronal function via increased expression of neurotransmitters such as substance P, calcitonin gene-related peptide (CGRP) and the like, showing low expression due to a diabetic condition.

While not limited to diabetic neuropathy, the basis of the management-treatment of diabetic complications is the maintenance of good control of blood glucose over a long term. However, a complete control of blood glucose in every-day medical care is impossible to perform, and establishment of a treatment method based on the aforementioned onset mechanism is necessary.

While AR inhibitors that suppress the activity of aldose reductase (AR), which is a rate-limiting enzyme of the polyol pathway, are used for every-day medical care, international consensus relating to their clinical usefulness has not been obtained. As a therapeutic drug capable of improving metabolic abnormalities other than polyol metabolism and even blood flow abnormalities, AR inhibitor is the most expected pharmaceutical agent. In addition, various pharmaceutical agents (prostaglandin E₁, prostaglandin I₂, niceritrol, cilostazol, sarpogrelate hydrochloride) having a blood flow improving effect have been used for many patients. As pharmaceutical agents expected to be therapeutic drugs in the future, PKC-beta inhibitors, nonenzymatic glycation reaction inhibitors, antioxidants, nerve growth factors (NGF) and the like can be mentioned. However, treatments with these pharmaceutical agents have not been established.

On the other hand, an effect on severe neuropathy accompanying irreversible tissue changes can no longer be expected by improving control of blood glucose or a treatment with a pharmaceutical agent based on etiology, and a symptomatic therapy of subjective symptoms such as pain and the like is required. For pain, salicylic acid and non-steroidal anti-inflammatory drug as analgesics become the basis of the treatment, and administration of anticonvulsants such as indomethacin suppositories, carbamazepine and phenytoin has been employed for severe pain during the night. In addition, concurrent use of tricyclic anti-depressants and tetracyclic anti-depressants is said to be effective for resolution of symptomatic depression accompanying insomnia. Moreover, the effectiveness of mexiletine, which is an oral antiarrhythmic agent, has been elucidated.

In recent years, new oral hypoglycemic drugs and insulin preparations have appeared one after another, and maintenance of better blood glucose control has been enabled, which is expected to lead to the suppression of the onset and progress of diabetic complications including neuropathy. Nevertheless, such is not sufficient, and the establishment of a more effective method for the prophylaxis or treatment of diabetic complications has been desired.

Basic fibroblast growth factor (bFGF) is a cytokine having an angiogenesis action, and its effectiveness has been reported through a study using an ischemic model (WO94/27630). Furthermore, bFGF has been clarified to act on various cells such as fibroblast and neurocytes, as an inducer of growth and differentiation. There is also a report on the effectiveness of bFGF for the treatment of diabetic neuropathy (Nakamura et al., "Effect of bFGF on diabetic neuropathy", the 46th Japan Diabetes Convention, abstract, II-J3-28, May, 2003).

### Disclosure of the Invention

The problem to be solved by the present invention is provision of a pharmaceutical agent capable of more effectively treating diabetic complications.

The present inventors took note of the treatment effect of bFGF on diabetic neuropathy and, to more efficiently express its action and effect, conducted intensive studies and found that a desired object can be achieved by carrying bFGF on a gelatin gel, which resulted in the completion of the present invention. Accordingly, the present invention provides the following.
[1] A therapeutic agent for diabetic neuropathy, which comprises bFGF and a gelatin gel carrying the bFGF.
[2] The therapeutic agent of [1], wherein the gelatin gel is a crosslinked gelatin gel.
[3] A method of treating diabetic neuropathy comprising administering a therapeutically effective amount of bFGF carried on a gelatin gel to a mammal.
[4] The method of [3], wherein the gelatin gel is a crosslinked gelatin gel.
[5] Use of bFGF and a gelatin gel for the production of a therapeutic agent for diabetic neuropathy.
[6] The use of [5], wherein the gelatin gel is a crosslinked gelatin gel.
[7] A commercial package comprising the therapeutic agent of [1] or [2] and a written matter associated therewith, which states that the therapeutic agent can or should be used for diabetic neuropathy.

In the following, a therapeutic agent for diabetic neuropathy wherein bFGF is carried on a gelatin gel is also simply referred to as "the preparation of the present invention".

The present invention is useful in that carrying bFGF on a gelatin has afforded remarkably improved nerve conduction velocity and intraneural blood flow volume, that are the indices for the treatment of diabetic neuropathy, and improved utility of bFGF as a therapeutic agent for diabetic neuropathy. Using the preparation of the present invention, diabetic neuropathy can be treated effectively.

### Brief Description of the Drawings

Fig. 1 is a graph showing the nerve conduction velocity of the sciatic nerve at 10 days after the treatment when a crosslinked gelatin gel (control) or a bFGF-carrying crosslinked gelatin gel is intramuscularly injected into normal rat and STZ-induced diabetic rat.
Fig. 2 is a graph showing the intraneural blood flow volume of the sciatic nerve at 10 days after the treatment when a crosslinked gelatin gel (control) or a bFGF-carrying crosslinked gelatin gel is intramuscularly injected into normal rat and STZ-induced diabetic rat.

### Best Mode for Embodying the Invention

As the bFGF in the present invention, a homolog thereof may be used. The bFGF and/or its homolog in the present invention can be obtained by isolation and purification of a product from a microorganism or a culture cell naturally or by genetic recombination technique, or by chemical modification or biological modification thereof. As the bFGF to be used in the present invention, human bFGF or its homolog is particularly preferable.

The homolog of bFGF means a polypeptide of the following [I] or [II].
[I] A polypeptide containing an amino acid sequence substantially the same as bFGF produced by a mammal. The substantially same amino acid sequence means an amino acid sequence containing 1 to 6 amino acids substituted by different kind(s) of amino acid(s) and having a biological activity of bFGF.
[II] A polypeptide wherein additional amino acid segment(s) is(are) added to N-terminal and/or C-terminal of bFGF produced by a mammal, or N-terminal and/or C-terminal of the polypeptide of the above-mentioned [I]. The additional amino acid segment consists of 1 to 12 amino acids, which does not impair the biological activity of bFGF or the polypeptide of the above-mentioned [I].

While human bFGF is a polypeptide consisting of 146 amino acids, in the preparation of the present invention, for example, polypeptide consisting of 146 amino acids described in JP-A-2-504468 may be used as a homolog of human bFGF (homolog of the aforementioned [I]). In the polypeptide, the 69th cysteine (Cys) and the 87th cysteine (Cys) constituting the amino acid sequence of human bFGF are respectively substituted by serine (Ser).

Moreover, as a homolog of the aforementioned [II], for example, a polypeptide consisting of 155 amino acids described in JP-A-63-500843 may be used. In this polypeptide, a segment consisting of 9 amino acids is added to the N-terminal of human bFGF.

In addition, a polypeptide consisting of 147 amino acids wherein Met- is added to the N-terminal, or a polypeptide described in JP-A-63-501953, which consists of 157 amino acids wherein a segment consisting of 11 amino acids is added to the N-terminal may be used.

As a particularly preferable bFGF, Trafermin (genetic recombinant) can be used.

In the preparation of the present invention, one kind of bFGF may be used alone, or plural kinds thereof may be used in combination. Moreover, as mentioned above, while the homolog of bFGF includes plural kinds, such homologs may be used alone or in combination.

Since the residual amount of bFGF in the living organisms is an ultratrace amount, for commercially stable supply of the preparation of the present invention, bFGF produced by microorganisms such as *Escherichia coli* and the like or produced in a cultured cell by genetic recombination techniques or a homolog thereof is particularly preferably used. When a gene for producing bFGF or a homolog thereof (generally, polypeptide of the aforementioned [I] in this case) is incorporated into a microorganism or cultured cell, what is produced by the microorganism or cultured cell is generally a polypeptide wherein additional amino acid segment(s) is(are) added to N-terminal and/or C-terminal of bFGF, or N-terminal and/or C-terminal of the polypeptide of the above-mentioned [I], i.e., the polypeptide of the aforementioned [II].

The kind of the gelatin to be the starting material of the gelatin gel in the present invention is not particularly limited, and any that can be usually obtained may be used. As such gelatin, for example, an alkali-treated gelatin having an isoelectric point of around 5 (acidic gelatin), an acid-treated gelatin having an isoelectric point of around 9 (basic gelatin) and the like can be mentioned, with preference given to an acidic gelatin having an isoelectric point of around 5, from the aspect of affinity to bFGF. Not only one kind of gelatin but an appropriate mixture of gelatins having different physical properties such as starting material, solubility, molecular weight, isoelectric point and the like may be used.

While the crosslinking agent for crosslinking the gelatin usable in the present invention is not particularly limited as long as it is not toxic to a living body, for example, glutaraldehyde, water-soluble carbodiimide such as 1-ethyl-3-(3-dimethylaminopropyl)carbodiimide hydrochloride, 1-cyclohexyl-3-(2-morpholinoethyl)carbodiimide-metho-p-toluenesulfonate and the like, bisepoxy compound, formalin and the like are preferable, and glutaraldehyde and 1-ethyl-3-(3-dimethylaminopropyl)carbodiimide hydrochloride are particularly preferable.

Gelatin may also be crosslinked by a heat treatment, ultraviolet light irradiation or electron irradiation.

The form of the crosslinked gelatin gel to be used in the present invention is not particularly limited and, for example, the form of a cylinder, a prism, a sheet, a disk, spheres, particles, granules, a paste and the like can be mentioned. For use as an implant, the form of a cylinder, a prism, a sheet and a disk are preferable. For use as an injectable preparation, the form of spheres, particles, granules and a paste are preferable.

A crosslinked gelatin gel having the form of a cylinder, a prism, a sheet or a disk can be prepared by adding an aqueous solution of a crosslinking agent to an aqueous solution of gelatin, or adding gelatin to an aqueous solution of a crosslinking agent, casting the mixture into a mold having a desired form and allowing a crosslinking reaction. A molded gelatin gel may be used as it is, or an aqueous solution of a crosslinking agent may be added after drying. For quenching the crosslinking reaction, the reaction product is brought into contact with a low-molecular-weight substance having an amino group such as ethanolamine, glycine and the like, or an aqueous solution having not more than pH 2.5 is added. The obtained crosslinked gelatin gel is washed with distilled water, ethanol, 2-propanol (hereinafter to be referred to as IPA), acetone and the like and subjected to the formulation of a preparation.

The water content of the obtained crosslinked gelatin gel is 50-99 w/w%. Here, the water content of gel is a proportion of the weight of water in a gel relative to the whole weight of the gel when the gel is wet.

A crosslinked gelatin gel in the form of a paste can be prepared by a method similar to the preparation method of the aforementioned crosslinked gelatin gel in the form of a cylinder, a prism, a sheet or a disk.

A crosslinked gelatin gel in the form of spheres, particles or granules is obtained, for example, by charging an aqueous solution of gelatin in an apparatus equipped with a stirrer motor (e.g., Three-one motor, manufactured by SHINTO Scientific Co., Ltd., EYELA mini D.C. Stirrer etc.) and a stirring propeller of Teflon (trademark) in a three-necked round flask, adding an oil such as olive oil and the like, stirring the mixture at about 200-600 rpm to give a W/O emulsion and adding an aqueous solution of a crosslinking agent, or previously emulsifying an aqueous solution of gelatin in olive oil (e.g., vortex mixer Advantec TME-21, homogenizer polytron PT10-35 etc.), adding the emulsion dropwise to olive oil to give a fine particulate W/O emulsion, adding an aqueous solution of a crosslinking agent thereto to allow crosslinking reaction, recovering the crosslinked gelatin gel by centrifugation, washing the gel with acetone, ethyl acetate etc., further washing the gel with IPA, ethanol etc. and drying the gel. Then, 100 mM glycine is added to an aqueous solution containing Tween 80, and the particles are suspended therein to quench the crosslinking reaction. The obtained crosslinked gelatin gel in the form of particles are washed successively with IPA, distilled water containing Tween 80, distilled water etc. and subjected to the formulation of a preparation.

The average particle size of the obtained crosslinked gelatin gel particles is 1-1000 µm, where particles having a necessary size are sieved as appropriate depending on the object and used. For administration by intramuscular injection, particles having an average particle size of 10-150 µm are preferable. As used herein, the average particle size means an average diameter of the particles, which is assumed by passing the sieves. Moreover, the water content of the obtained crosslinked gelatin gel particles is about 50-99 w/w%, and particles having a preferable water content can be prepared as appropriate. As used herein, the water content of gel means a proportion of the weight of water in a gel relative to the whole weight of the gel when the gel is wet.

When the crosslinked gelatin gel particles are coagulated, for example, ultrasonication (within about 1 min under cooling is preferable) and the like may be applied. By pre-emulsifying, moreover, crosslinked gelatin gel in the form of fine particles having an average particle size of not more than 20 µm can be obtained.

As a different method of preparing the crosslinked gelatin gel in the form of spheres or particles, the following method can be mentioned.

Olive oil is charged in an apparatus in the same manner as in the above-mentioned method, the mixture is stirred at about 200-600 rpm, an aqueous solution of gelatin is added dropwise thereto to give a W/O emulsion, the emulsion is cooled, acetone, ethyl acetate and the like are added, the mixture is stirred and gelatin particles are recovered by centrifugation. The recovered gelatin particles are futher washed with acetone, ethyl acetate etc., and then with IPA, ethanol etc. and dried. The dry gelatin particles are suspended in an aqueous solution of a crosslinking agent containing 0.1% Tween 80, and the mixture is gently stirred to cause a crosslinking reaction, and the crosslinking reaction is quenched by washing with a 100 mM glycine aqueous solution containing 0.1% Tween 80, 0.004N HCl containing 0.1% Tween 80 or the like, depending on the crosslinking agent used, whereby crosslinked gelatin gel particles can be obtained.

The average particle size and water content of the crosslinked gelatin gel particles obtained by this different method are the same as those obtained by the above-mentioned method.

While the conditions for the crosslinking reaction should be appropriately determined, the reaction temperature is preferably 0-40°C, and the reaction time is preferably 1-48 hr.

The crosslinked gelatin gel obtained as mentioned above can also be vacuum dried or freeze dried.

For freeze-drying, for example, a crosslinked gelatin gel is placed in distilled water, frozen in liquid nitrogen for not less than 30 min or at -80°C for not less than 1 hr and dried in a freeze dryer for 1 to 3 days.

While the concentrations of the gelatin and a crosslinking agent for preparation of a crosslinked gelatin gel should be appropriately determined depending on the desired water content, the gelatin concentration is preferably 1-100 w/v%, and the concentration of the crosslinking agent is preferably 0.01-100 w/v% (corresponding to 1-5400 mM).

The crosslinked gelatin gel can be made to have a desired water content by changing the concentrations of gelatin (starting material) and the crosslinking agent. To increase the water content, both the gelatin concentration and the crosslinking agent concentration need to be decreased and, conversely, when the water content is decreased, both the gelatin concentration and the crosslinking agent concentration need to be increased.

To carry bFGF on the crosslinked gelatin gel prepared as mentioned above, a bFGF aqueous solution may be added dropwise to the crosslinked gelatin gel to cause impregnation, or the crosslinked gelatin gel may be suspended in an aqueous solution of bFGF to cause re-swelling.

While the amount of bFGF that can be carried on a crosslinked gelatin gel varies depending on the water content and the like of the crosslinked gelatin gel, it is 0.1-500 µg per 1 mg of the crosslinked gelatin gel.

The release time and release amount of bFGF from the gelatin gel vary depending on various conditions including the water content of the gelatin gel, the physical property of the gelatin used, such as isoelectric point and the like, the amount of bFGF to be carried in the preparation, the site to be administered to and the like.

The bFGF-carrying crosslinked gelatin gel preparation obtained as mentioned above can also be freeze-dried. For freeze-drying, for example, the preparation is frozen in liquid nitrogen for not less than 30 min or at -80°C for not less than 1 hr and dried in a freeze dryer for 1 to 3 days.

When the preparation of the present invention is to be formulation into an injectable preparation, the preparation is appropriately suspended in a medium such as purified water for injection, saline, buffer and the like. As the buffer, phosphate buffer, acetate buffer, citrate buffer and the like can be mentioned. Where necessary, dispersing agents, surfactants, isotonicity agents, pH adjusting agents, soothing agents, stabilizers, preservatives, coloring agents and the like, which are generally used for the production of an injectable preparation, can be appropriately added.

The preparation of the present invention has a superior diabetic neuropathy healing action, and can be used for the treatment of diabetic neuropathy. In addition, the preparation of the present invention is applicable to the treatment of diabetic neuropathy not only in human but also in other mammals (e.g., mouse, rat, hamster, rabbit, cat, dog, bovine, sheep, monkey and the like).

The diabetic neuropathy in the present invention means degraded action of the peripheral nerve due to hyperglycemia. While diabetic neuropathy is classified into polyneuropathy (e.g., numbness, chill, neuralgia, sensory paralysis, calf cramps and the like), autonomic neuropathy (e.g., sweating abnormality, lightheadedness, constipation, diarrhea, degraded gallbladder contractile ability and the like), mononeuropathy (e.g., paralysis of facial nerve, extraocular muscles or acoustic nerve, limbs neuropathy and the like) and the like, the preparation of the present invention can be applied to any of these conditions. Preferably, it is applicable to polyneuropathy.

While the preparation of the present invention can be administered by any administration route such as intravenous administration, intramuscular administration and the like, intramuscular administration is preferable. For example, bFGF-carrying particulate gel is suspended in a suitable medium conventionally used for the production of injection to give an injectable preparation and the preparation is intramuscularly injected. In addition, bFGF-carrying gelatin gel may be formed into a cylinder, a prism, a sheet, a disk and the like and embedded in a neuropathic part.

While it is difficult to specify the dose of the therapeutic agent of the present invention since it varies depending on the kind of the target and indication, the level of condition, and the age and pathology of subject, for example, in the case of treatment of diabetic neuropathy in human, it is within the range of about 0.001 µg - 10 mg, preferably 1 - 1000 µg in the amount of bFGF, per one treatment site for one treatment. While the administration frequency varies depending on the cases and the dose for one treatment, it is generally about 1 to 10 times. Depending on the kind and level of condition, the therapeutic agent may be administered 2 to 6 times.

### Examples

The present invention is explained in detail in the following by referring to Examples, which are not to be construed as limitative.

### Preparation of crosslinked gelatin gel (water content 99%)

An aqueous solution of glutaraldehyde was added to an aqueous solution of gelatin (alkali-treated gelatin having isoelectric point around 5 (type B, manufactured by Nippi, Inc.)), the mixture was cast in a cylindrical mold to cause a crosslinking reaction, whereby a crosslinked gelatin gel was prepared. The crosslinked gelatin gel was immersed in a glycine aqueous solution to quench the crosslinking reaction, then washed by immersion in water for injection, and freeze-dried to give a crosslinked gelatin gel.

### Preparation of injection

To the above-mentioned freeze-dried crosslinked gelatin gel was added a bFGF aqueous solution having a concentration of 50 µg/800 µL to allow impregnation with bFGF, whereby an injection (bFGF-carrying crosslinked gelatin gel) was prepared.

### Experiment method

Streptozotocin (STZ) was dissolved in sterile saline to a final concentration of 0.9% and the solution was intraperitoneally administered to 8-week-old male Wistar rat at 60 mg per 1 kg body weight to prepare a diabetic rat. A bFGF (50 µg)-carrying crosslinked gelatin gel (800 µL) was divided into quarters and intramuscularly administered to untreated normal rat and diabetic rat at 8 weeks after STZ administration in the right femoral muscle and soleus muscle, 2 sites each, totaling 4 sites (12.5 µg/0.2 mL x4). As a control, a crosslinked gelatin gel not carrying bFGF was intramuscularly administered to the left femoral muscle and soleus muscle. At 10 days after the treatment, fundus photography and retina blood flow measurement were performed. The nerve conduction velocity was obtained by electrically stimulating from the sciatic notch and Achilles tendon, receiving the stimulus at the plantar part, measuring each conduction time using Neuropack ERECTROMYOGRAPH MEM-3202, then measuring the distance between stimulation sites, and calculating from the distance (distance between two stimulation points)/conduction time (sciatic notch-Achilles tendon). The intraneural blood flow volume was measured by puncturing an electrode needle into the sciatic nerve, generating hydrogen there by electrolysis, and measuring the hydrogen clearance by semiconductor laser tissue blood flow meter MODEL LBF-221R SERIAL 91052.

### Results

In the funduscopy, the onset of retinopathy was not observed in any group. While the retinal blood flow volume was found to have decreased in diabetic rat, bFGF administration did not cause any changes.

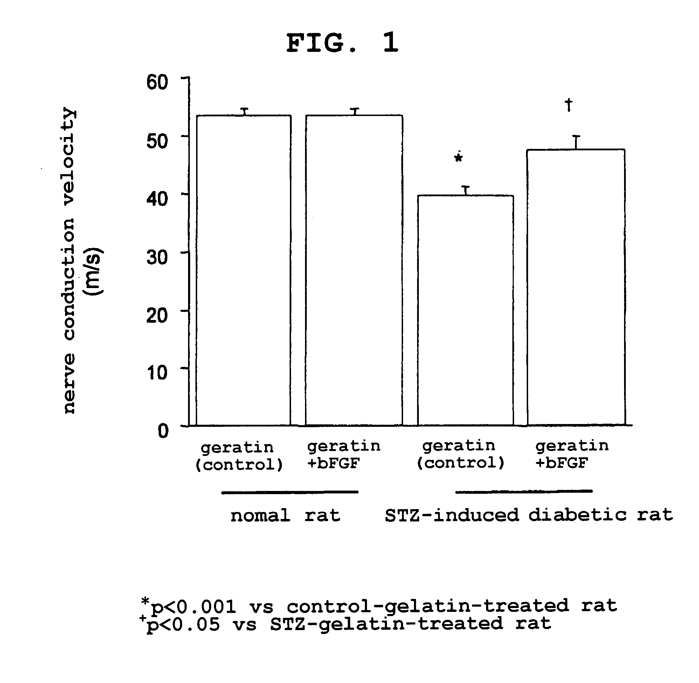

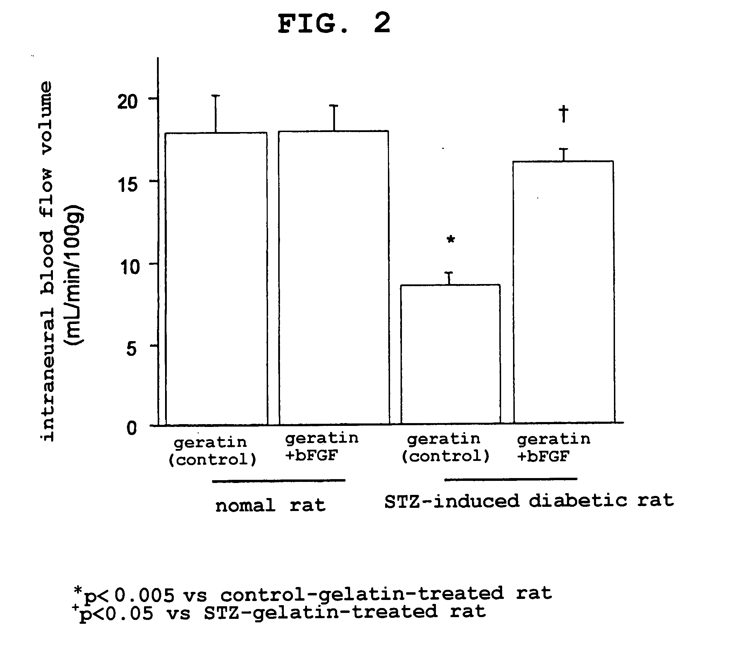

The measurement results of the nerve conduction velocity are shown in Fig. 1 and the measurement results of the intraneural blood flow volume are shown in Fig. 2.

From the results of Fig. 1 and Fig. 2, it is appreciated that the administration of the bFGF-carrying gelatin preparation did not affect nerve conduction velocity and intraneural blood flow volume in normal rat, but significantly improved decreased nerve conduction velocity and decreased intraneural blood flow volume, caused by diabetes, in diabetic rat alone.

From the foregoing results, it has been clarified that the preparation of the present invention can effectively treat diabetic neuropathy.

### Industrial Applicability

The present invention is useful in that remarkable improvement in the nerve conduction velocity and intraneural blood flow volume to be the indices in the treatment of diabetic neuropathy can be afforded by carrying bFGF on gelatin, and the utility of bFGF as a therapeutic agent for diabetic neuropathy has been improved. With the preparation of the present invention, diabetic neuropathy can be effectively treated.

While some of the embodiments of the present invention have been described in detail in the above, it will, however, be clear for those of ordinary skill in the art that various modifications and changes may be made to the particular embodiments shown without substantially departing from the teaching and advantages of the present invention. Such modifications and changes are encompassed in the spirit and scope of the present invention as set forth in the appended claims.

This application is based on a patent application No. 2004-116109 filed in Japan (filing date: April 9, 2004), the contents of which are incorporated in full herein by this reference.

## Claims

1. A therapeutic agent for diabetic neuropathy, which comprises a basic fibroblast growth factor and a gelatin gel carrying the basic fibroblast growth factor.

2. The therapeutic agent of claim 1, wherein the gelatin gel is a crosslinked gelatin gel.

3. A method of treating diabetic neuropathy comprising administering a therapeutically effective amount of a basic fibroblast growth factor carried on a gelatin gel to a mammal.

4. The method of claim 3, wherein the gelatin gel is a crosslinked gelatin gel.

5. Use of a basic fibroblast growth factor and a gelatin gel for the production of a therapeutic agent for diabetic neuropathy.

6. The use of claim 5, wherein the gelatin gel is a crosslinked gelatin gel.

7. A commercial package comprising the therapeutic agent of claim 1 or claim 2 and a written matter associated therewith, which states that the therapeutic agent can or should be used for diabetic neuropathy.
